# EUROPEAN PATENT APPLICATION

(11) **EP 2 671 500 A2**
(43) Date of publication of application: **11.12.2013**
(21) Application number: 13168858.2
(22) Date of filing: 23.05.2013
(51) Int. Cl.: A61B 1/00, A61B 1/06

(54) **Endoscope and endoscope system**

(30) Priority: 05.06.2012 JP 2012128135
(71) Applicant: Canon Kabushiki Kaisha, Tokyo 146-8501 (JP)
(72) Inventor: Hayama, Akira, Tokyo,, Tokyo 146-8501 (JP)
(74) Representative: TBK

(57) **Abstract**

An endoscope has an illuminating-light exit portion (24) that allows illuminating light for illuminating an object to exit, and an observing portion (23) that captures an image of the object. The endoscope includes changing means (13) configured to change a light quantity distribution of the illuminating light. The changing means changes the light quantity distribution of the illuminating light based on information of the image captured by the observing portion.

## Description

### BACKGROUND OF THE INVENTION

### Field of the Invention

The present application relates to an endoscope used for observing the interior of a human body or a structure that cannot be directly viewed by humans, and also relates to an endoscope system including the endoscope.

### Description of the Related Art

In the related art, illuminating devices for endoscopes are designed to uniformly illuminate an object with illuminating light exiting from a tip of an insertion portion. In other words, such illuminating devices illuminate an object so as not to create a contrast between bright and dark on the object.

Such illuminating devices for uniform illumination do not create shadows even when there are slight irregularities in an affected area in the center of an image. This makes it difficult to discover such irregularities and to diagnose the extent of the irregularities.

Japanese Patent Laid-Open No. 2007-021002 describes a method for facilitating stereoscopic viewing by producing an imbalance in illumination distribution to create shadows in an affected area.

Japanese Patent Laid-Open No. 2012-075658 describes an endoscope in which an observation window protrudes from an end portion of an insertion portion of the endoscope in the axial direction thereof, and an illumination window is provided in an inclined surface.

Findings of the present inventors indicate that, in operation of an endoscope, if the orientation or position of the endoscope is slightly changed after illumination for an object is optimized, it may be difficult to observe the object.

In an endoscope described in Japanese Patent Laid-Open No. 2007-021002, an imbalance is created in the illumination distribution to optimize illumination such that the quantity of illuminating light is non-uniformly distributed. However, it has been found that if the orientation of the endoscope is changed, the illumination needs to be optimized again.

In particular, if the endoscope has an illumination window in an inclined surface, as in the case of the endoscope described in Japanese Patent Laid-Open No. 2012-075658, a change in orientation of the endoscope causes a significant change in the state of illumination.

### SUMMARY OF THE INVENTION

The present disclosure provides an endoscope and an endoscope system with which, when the orientation or position of the endoscope is changed by moving or rotating the endoscope, it is possible to easily correct the illumination distribution and easily observe an object.

The present invention in its first aspect provides an endoscope as specified in Claims 1 to 13.

The present invention in its second aspect provides an endoscope system as specified in Claim 14.

The present invention in its third aspect provides a method for illuminating an object with an endoscope as specified in Claim 15.

Further features of the present invention will become apparent from the following description of exemplary embodiments with reference to the attached drawings.

### BRIEF DESCRIPTION OF THE DRAWINGS

Fig. 1 is a diagram for explaining an endoscope according to a first embodiment.

Fig. 2A to Fig. 2E are diagrams for explaining an operation of the endoscope according to the first embodiment.

Fig. 3 illustrates a processing flow of the endoscope according to the first embodiment.

Fig. 4 is a diagram for explaining captured images of an object.

Fig. 5 is a diagram for explaining a configuration of an endoscope system according to a second embodiment.

Fig. 6 is a diagram illustrating a tip of an endoscope according to the second embodiment.

Fig. 7 is a diagram illustrating a tip of an endoscope according to a third embodiment.

Fig. 8 is a functional block diagram for explaining a configuration of the endoscope according to the third embodiment.

### DESCRIPTION OF THE EMBODIMENTS

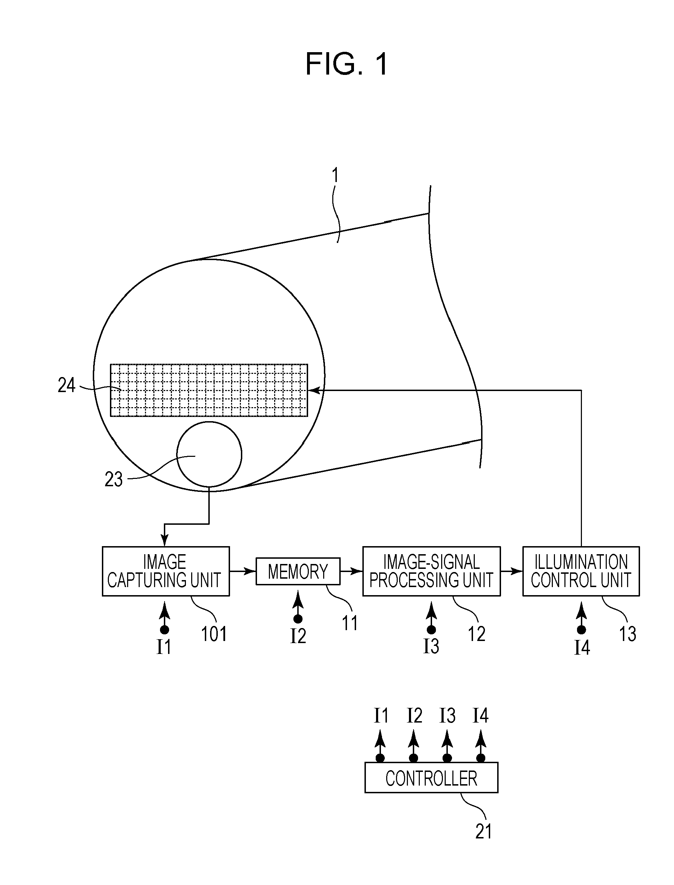

As illustrated in Fig. 1, in an endoscope according to a first embodiment, an endoscope tip 1 serving as an insertion portion has an observing portion 23 and an illuminating-light exit portion 24.

The observing portion 23 captures optical information serving as image information of an object. The observing portion 23 is formed by an imaging optical system including an objective lens, optical fibers, and a light transmitting window for observation. An image of an object is captured by an image pickup element provided inside or outside an endoscope main body. Alternatively, the observing portion 23 may include an image pickup element, such as a semiconductor sensor, so as to capture an image.

The illuminating-light exit portion 24 is formed by an illumination optical system including a lens, optical fibers, and an illuminating-light transmitting window. The illuminating-light exit portion 24 illuminates an object with light from a light source provided inside or outside the endoscope main body. Alternatively, the illuminating-light exit portion 24 may include a light modulating element, such as a liquid crystal element or an electrochromic (EC) element, or a light emitting element, such as a light emitting diode (LED) array, so as to generate illuminating light having a desired light quantity distribution. To generate light having a desired light quantity distribution, a mechanical mechanism, such as an aperture or a shutter, may be used instead of the electronic element described above.

The shapes of the observing portion 23 and the illuminating-light exit portion 24 in plan view are not limited to circular and rectangular shapes, but may be shapes formed by appropriate straight and/or curved lines. Specifically, the observing portion 23 and the illuminating-light exit portion 24 may have a semicircular shape, an oval shape, or a polygonal shape, such as a triangular shape, a pentagonal shape, a hexagonal shape, or an octagonal shape, in plan view.

As an example of the endoscope illustrated in Fig. 1, a configuration will be described, in which the observing portion 23 is formed by a circular objective lens and the illuminating-light exit portion 24 is formed by a rectangular liquid crystal element having a plurality of pixels that transmit illuminating light.

The endoscope includes an image capturing unit 101, a memory 11, an image-signal processing unit 12, and an illumination control unit 13. The operations of the image capturing unit 101, the memory 11, the image-signal processing unit 12, and the illumination control unit 13 are controlled in accordance with control information signals I1 to I4 from a controller 21.

The illuminating-light exit portion 24 is turned on by the illumination control unit 13 and illuminates an object. Light reflected from the object passes through the observing portion 23 and forms an image of the object onto a semiconductor image sensor within the image capturing unit 101, so that an image signal is generated.

The generated image signal of the object is temporarily stored in the memory 11, such as a semiconductor memory. The stored image signal is subjected to extraction of feature points of the image in the image-signal processing unit 12.

Next, the orientation of the endoscope is changed to capture another image. Again, the resulting image signal is stored in the memory 11 and subjected to extraction of feature points in the image-signal processing unit 12.

On the basis of the extracted feature points, matching is performed on image signals of at least two frames sequentially captured. This is to detect a position within a field of view (i.e., within a scope) to which a specific region of the object, whose images have been captured, has been moved by changing the orientation of the endoscope.

In accordance with the result of the detection, the illumination control unit 13 drives pixels of the liquid crystal element, which is the illuminating-light exit portion 24, such that the specific region is illuminated at an appropriate intensity.

Then, the object is illuminated with light having a desired illuminating-light intensity distribution, and an image of the object is captured again. That is, a first intensity distribution of illuminating light for image capturing before the orientation of the endoscope tip 1 is changed and a second intensity distribution of illuminating light for image capturing after the orientation of the endoscope tip 1 is changed are made different from each other.

This makes it possible to acquire at least an image of one frame captured by illuminating the object with light having the first intensity distribution, at least an image of one frame captured by illuminating the object with light having the first intensity distribution after the orientation of the endoscope tip 1 is changed, and at least an image of one frame subsequently captured by illuminating the object with light having the second intensity distribution.

In the present embodiment, where a state of illumination is changed on the basis of information of an image previously captured, the object can be easily observed even if the orientation or position of the endoscope is changed by moving or rotating the endoscope.

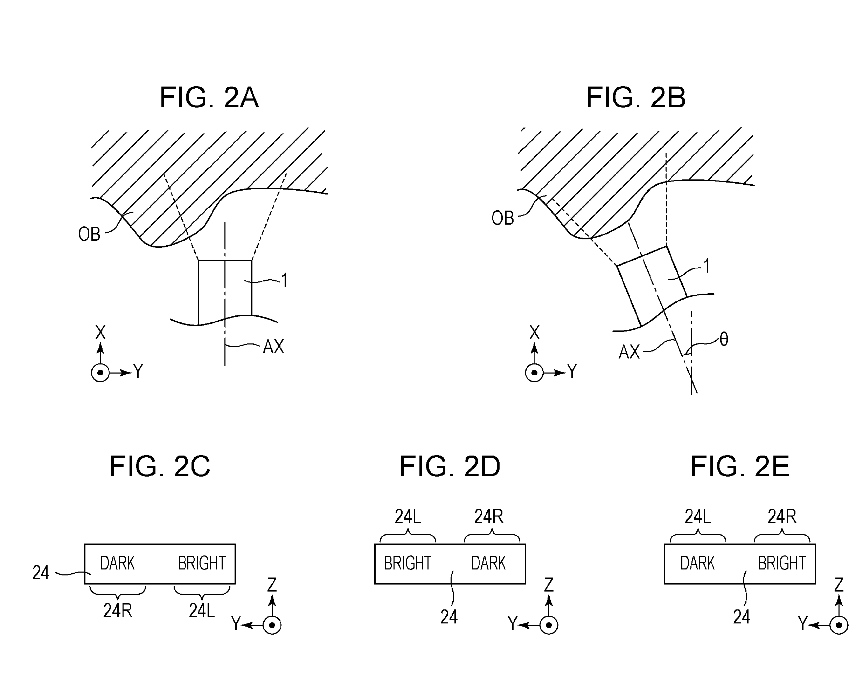

An effect achieved by the technique of varying the intensity distribution of illuminating light will now be described using examples. Fig. 2A to Fig. 2E schematically illustrate illumination and orientations of the endoscope. Fig. 2A and Fig. 2B each illustrate an orientation of the endoscope tip 1. Fig. 2C to Fig. 2E each are a front view of the illuminating-light exit portion 24, and each illustrate a light quantity distribution of light exiting from the illuminating-light exit portion 24.

As illustrated in Fig. 2A, in space coordinates, an object OB is observed in a state where an X axis and an optical axis AX are parallel to each other. The liquid crystal element is driven such that the object OB is illuminated with illuminating light having a non-uniform light intensity distribution that facilitates identification of microscopic irregularities of the object OB.

For example, in Fig. 2A, a protruding portion and its vicinity on the left side of the object OB are illuminated at a high intensity, whereas a recessed portion and its vicinity on the right side of the object OB are illuminated at a low intensity. For this, as illustrated in Fig. 2C, light exiting from the illuminating-light exit portion 24 is one having a light quantity distribution where a right side 24R of the illuminating-light exit portion 24 is dark and a left side 24L of the illuminating-light exit portion 24 is bright.

Next, as illustrated in Fig. 2B, the endoscope tip 1 is rotated by 180 degrees about the optical axis AX, which is inclined by an angle θ.

The relative position of the observing portion 23 and the illuminating-light exit portion 24 in the endoscope tip 1 is fixed. Therefore, when the object OB is observed without changing the driven state of the liquid crystal element (i.e., without changing the exit intensity distribution of illuminating light), the protruding portion and its vicinity on the left side of the object OB are illuminated at a low intensity, whereas the recessed portion and its vicinity on the right side of the object OB are illuminated at a high intensity.

In this case, as illustrated in Fig. 2D, light exiting from the illuminating-light exit portion 24 is one having a light quantity distribution where the right side 24R of the illuminating-light exit portion 24 is dark and the left side 24L of the illuminating-light exit portion 24 is bright.

Since not only the image of the object OB is rotated but also the intensity distribution of illuminating light is inverted, the way the object OB is viewed is changed significantly. This makes it difficult to identify the extent of microscopic irregularities of the object OB.

In the endoscope of the present embodiment, the rotation of a captured image is detected on the basis of feature points of the captured image. Then, the driven state of the liquid crystal element is changed to change the exit intensity distribution of illuminating light.

In this case, the intensity distribution is inverted 180 degrees. In other words, the liquid crystal element is driven to illuminate the object OB such that even if the illuminating-light exit portion 24 is physically inverted, the left side of the object OB is illuminated at a high intensity and the right side of the object OB is illuminated at a low intensity.

In this case, as illustrated in Fig. 2E, light exiting from the illuminating-light exit portion 24 is one having a light quantity distribution where the right side 24R of the illuminating-light exit portion 24 is bright and the left side 24L of the illuminating-light exit portion 24 is dark.

Moreover, since the orientation of the endoscope tip 1 (i.e., the optical axis AX) is inclined by the angle θ, this angle of inclination is detected from the captured image as necessary. Then, the liquid crystal element is driven to realize an exit intensity distribution appropriate for the current state.

The operation of driving the liquid crystal element can be controlled by outputting, from the illumination control unit 13 to the liquid crystal element, a driving signal for determining a light transmitting state of each pixel of the liquid crystal element. Specifically, the liquid crystal element may be driven to slightly lower the intensity on the right side in Fig. 2B. This makes the brightness of the right side 24R in Fig. 2E slightly lower than that of the left side 24L in Fig. 2C.

The present disclosure may be configured such that after an intensity distribution of illuminating light is automatically changed on the basis of information of a captured image, the operator can manually change the intensity distribution through fine adjustments while viewing the captured image displayed on a display device.

In this case, the operator may input a change instruction with an input device, such as a pointer.

The image-signal processing unit 12 used in the present disclosure may include a display control circuit that generates a display image signal for displaying a captured image of an object on a display unit (not shown), a feature-point extracting means that extracts feature points from the image, and a tracking means that tracks the extracted feature points.

With the tracking means, it is possible to track feature points of an image, and thus to automatically change and optimize the illuminating condition, which is an image capturing condition, in accordance with a change in position of the endoscope tip 1.

As described in detail, the endoscope of the present application includes the illuminating-light exit portion 24 that allows illuminating light for illuminating the object OB to exit, the observing portion 23 that captures an image of the object OB, and the illumination control unit 13 that serves as a changing means for changing a light quantity distribution of the illuminating light.

The endoscope has a first illuminating mode (see Figs. 2A and 2C) in which the object OB is illuminated with illuminating light having a first light quantity distribution when the endoscope tip 1 is located at a first position, and a second illuminating mode (see Figs. 2B and 2E) in which the object OB is illuminated with illuminating light having a second light quantity distribution different from the first light quantity distribution when the endoscope tip 1 is located at a second position different from the first position.

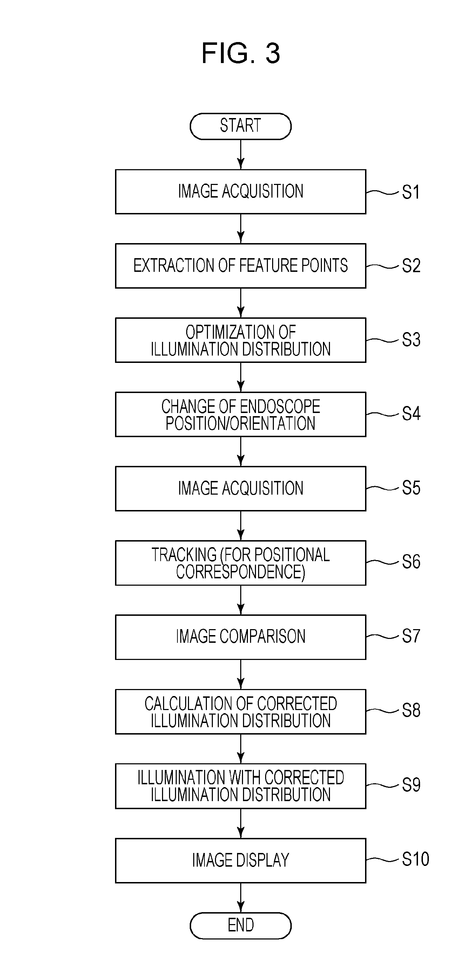

With reference to the flowchart of Fig. 3, an illuminating method of the endoscope according to the first embodiment will now be described.

### (Image Acquisition)

In image acquisition step S1, the operator inserts the endoscope tip 1 into a body, and illuminates an object with light having a uniform intensity distribution. Then, the image capturing unit 101 of the endoscope captures an image of the object. The captured image is stored in the memory 11.

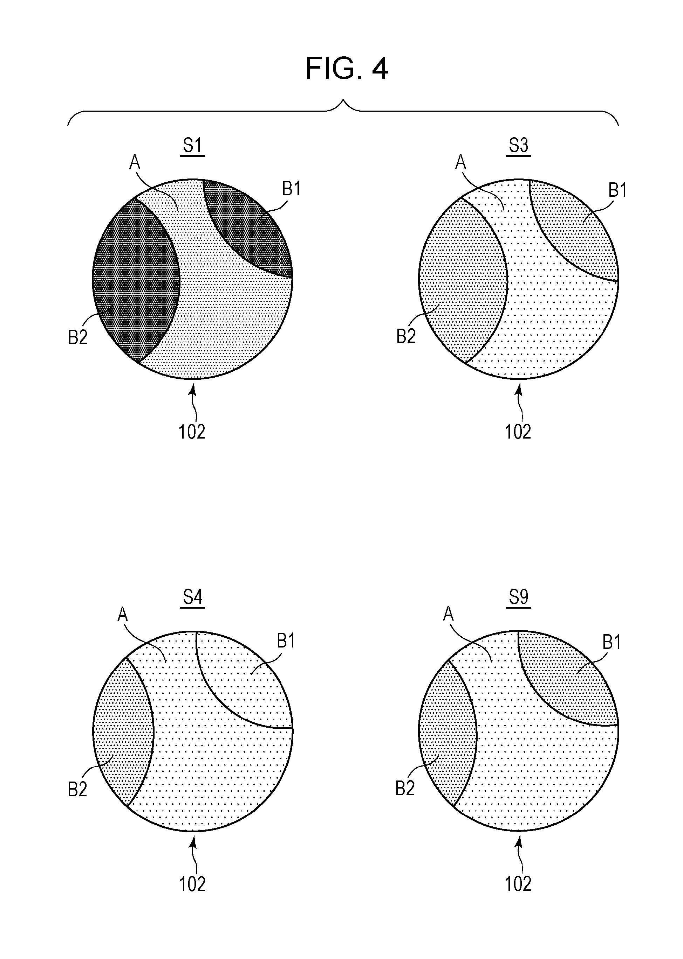

State S1 of Fig. 4 is a state of an observation image 102, which is an image of an object within an observation field of view, the image being captured and stored in the memory 11. Region A and regions B1 and B2 of Fig. 4 show different optical states, such as those of different tissues.

In the observation image 102 in state S1 of Fig. 4, region A is brightest, and regions B1 and B2 are almost completely dark and do not allow identification of surface conditions of an area to be observed.

### (Extraction of Feature Points)

In feature-point extraction step S2 of Fig. 3, an image signal acquired in image acquisition step S1 is analyzed to extract feature points that can be used as markers for tracking.

Feature points may be automatically extracted and set by a control circuit (not shown) on the basis of image analysis. Alternatively, setting information of feature points may be manually set by the operator using an input device, such as a touch panel (not shown).

The present embodiment involves performing a connected-region detection process, after binarization, to extract boundaries where there is a large contrast between bright and dark regions. Then, the extracted boundaries are used as feature points. In state S1 of Fig. 4, a boundary between regions A and B1 and a boundary between regions A and B2 are used as feature points.

There are other ways for the operator to set feature points. If there are no appropriate feature points in the object, the operator may input feature points with an input device, such as a pointer.

If a difference calculation is performed on the entire image for tracking, feature-point extraction step S2 of Fig. 3 may be eliminated.

As for a region where feature points are to be set, the operator may set a region of interest (ROI), within which feature points are extracted as described above.

### (Optimization of Illumination Distribution)

In illumination-distribution optimization step S3 of Fig. 3, an illumination distribution (i.e., a brightness distribution of an acquired image) is derived from the image acquired in image acquisition step S1. Then, the illumination distribution is changed such that regions B1 and B2 are illuminated at an intensity which allows identification of their surface conditions, and region A is illuminated at an intensity which does not cause brightness saturation and allows identification of surface conditions of region A.

For example, the transmittance of a group of pixels in the center of the liquid crystal element may be reduced to a level lower than that in step S1, and the transmittance of groups of pixels at right and left end portions of the liquid crystal element may be increased to a level higher than that in step S1. Thus, the state of illumination by the illumination optical system is set such that state S3 of Fig. 4 is realized.

State S3 of Fig. 4 is a state where the illumination distribution is optimized to facilitate not only the observation of region A and regions B1 and B2, but also the identification of boundaries between region A and regions B1 and B2.

For adjustment, a control table may be prepared which associates the brightness distribution of an image with the illumination distribution of illuminating light. Thus, the control circuit refers to the control table to perform control such that the illumination control unit 13 executes illumination correction.

Alternatively, the control of the illumination distribution may be expressed as a function depending on the illumination distribution. For example, a function may be prepared which multiplies a ratio between target and current illumination levels by a coefficient, so that the control circuit performs computing to execute illumination correction.

### (Change of Endoscope Position)

The change of the endoscope position in step S4 of Fig. 3 includes a translation in any direction, a change of orientation (or angle of inclination), and rotation.

In the endoscope disclosed herein, the endoscope tip 1 can be inclined in the range of 0 degrees to 180 degrees, more preferably in the range of 10 degrees to 100 degrees, with respect to the base of the endoscope. Also, the endoscope tip 1 can be rotated in the range of 0 degrees to 360 degrees, more preferably in the range of 0 degrees to 60 degrees, about the long axis of the endoscope.

When the orientation of the endoscope tip 1 is changed, the state of the observation image 102 changes from state S3 of Fig. 4 to state S4 of Fig. 4. The illumination distribution, which is in an optimal state in state S3 of Fig. 4, may not necessarily be optimal after the orientation of the endoscope tip 1 is changed.

Therefore, image acquisition is performed again to correct the illumination distribution such that observation or image capturing is performed in a state similar to state S3 of Fig. 4.

### (Image Acquisition)

Image acquisition step S5 of Fig. 3 equivalent to image acquisition step S1 is performed. The captured image is in state S4 of Fig. 4. State S4 differs from state S3 of Fig. 4 in the following ways.

In state S4 of Fig. 4, the brightness of region B1 is substantially the same as that of region A. This makes it difficult to identify the boundary between them. Also, the position of the object within the observation field of view in state S4 is slightly different from that in state S3.

As a result, the illumination distribution set in illumination-distribution optimization step S3 of Fig. 3 is no longer optimal at this point.

To make the illumination distribution close to an optimal illumination distribution, a change from the acquired image in state S3 of Fig. 4 to the acquired image in state S4 of Fig. 4 is detected and the illumination distribution is corrected in accordance with the detected change. The feature points extracted in feature-point extraction step S2 of Fig. 3 may be used to detect the change.

### (Tracking)

In step S6 of Fig. 3, feature points are extracted again by the same processing as that in feature-point extraction step S2. Thus, information of feature points extracted before and after the endoscope tip 1 is moved is obtained. This information is compared so as to establish a positional correspondence between images of the object captured before and after the endoscope tip 1 is moved.

### (Image Comparison)

In step S7 of Fig. 3, on the basis of the result of the tracking, an image comparison is made to compare brightness levels for the same region of the object.

As compared to the image in state S3 of Fig. 4, the brightness of the entire image in state S4 of Fig. 4 is higher, and a difference in brightness between regions A and B1 and a difference in brightness between regions A and B2 are smaller in state S4 of Fig. 4.

That is, the brightness of region A in state S4 of Fig. 4 is slightly higher than that of region A in state S3 of Fig. 4, and the degrees of increase in brightness (i.e., the change rates of brightness) of regions B1 and B2 are greater than the degree of increase in brightness (i.e., the change rate of brightness) of region A.

### (Calculation of Corrected Illumination Distribution)

In step S8 of Fig. 3, on the basis of the image comparison described above, a corrected illumination distribution is calculated to make the illumination distribution close to that in step S3 of Fig. 3. The same method as that used in optimizing the illumination distribution in step S3 can be used to calculate the corrected illumination distribution.

The corrected illumination distribution calculated and derived in the present embodiment is one which slightly lowers the brightness of region B1 to produce a difference in brightness between regions B1 and A.

The corrected illumination distribution calculated here may be applied to the entire image within the observation field of view. Alternatively, the operator may set an ROI in the image so that only the ROI is illuminated with the corrected illumination distribution.

### (Illumination with Corrected Illumination Distribution)

In step S9 of Fig. 3, the object is illuminated with light having the corrected illumination distribution calculated in step S8.

The illumination distribution is controlled by the illumination control unit 13. The illumination control unit 13 does not necessarily have to be a driving integrated circuit (IC) for driving the liquid crystal element. Specifically, the illumination control unit 13 may be provided in the path of the illumination optical system, and serve as an element, such as an aperture, for blocking part of light.

### (Image Display)

State S9 of Fig. 4 is a state of an image captured by illuminating the object with light having a corrected light intensity distribution in step S9 of Fig. 3. The image is displayed on a display device (not shown).
The corrected illumination distribution produces a difference in brightness and makes it easier to distinguish between regions A and B1.

Thus, a difference between regions A and B1 and a difference between regions A and B2 can be easily identified. This facilitates observation with the endoscope and eases the burden on the operator.

In the operator's operation, steps S1 to S10 of Fig. 3 are repeated. In the processing flow illustrated in Fig. 3, an acquired image is displayed on the display device only in step S10. Alternatively, an acquired image may also be displayed on the display device after step S5 and before step S9.

Even when the orientation of the endoscope tip 1 is changed, the illumination distribution is adjusted automatically. This makes it possible to carry out immediate observation and treatment.

As described in detail, a method for illuminating an object with the endoscope according to the present disclosure includes a first illuminating step of illuminating the object with illuminating light having a first light quantity distribution when the endoscope tip 1 is located at a first position (step S3 of Fig. 3), and a second illuminating step of illuminating the object with illuminating light having a second light quantity distribution different from the first light quantity distribution when the endoscope tip 1 is located at a second position different from the first position (step S9 of Fig. 3).

### Endoscope System

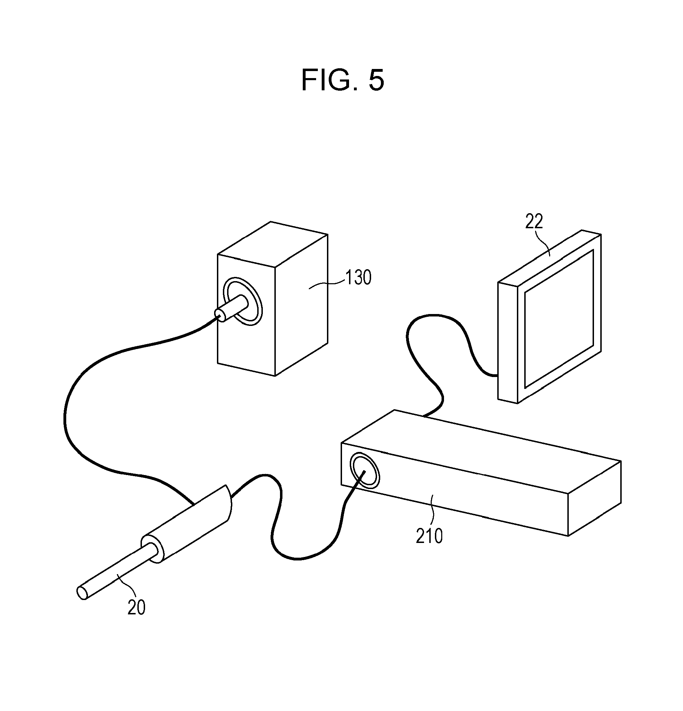

An endoscope system according to a second embodiment will now be described with reference to Fig. 5.

A control device 210 and a light source 130 are disconnectably connected to an endoscope main body 20. A display device 22 is connected to the control device 210.

The endoscope system illustrated in Fig. 5 is configured such that an object can be illuminated with light from the endoscope tip 1 (i.e., an insertion portion of the endoscope) through a light guide (not shown). The light guide is included in the endoscope and optically connected to the light source 130.

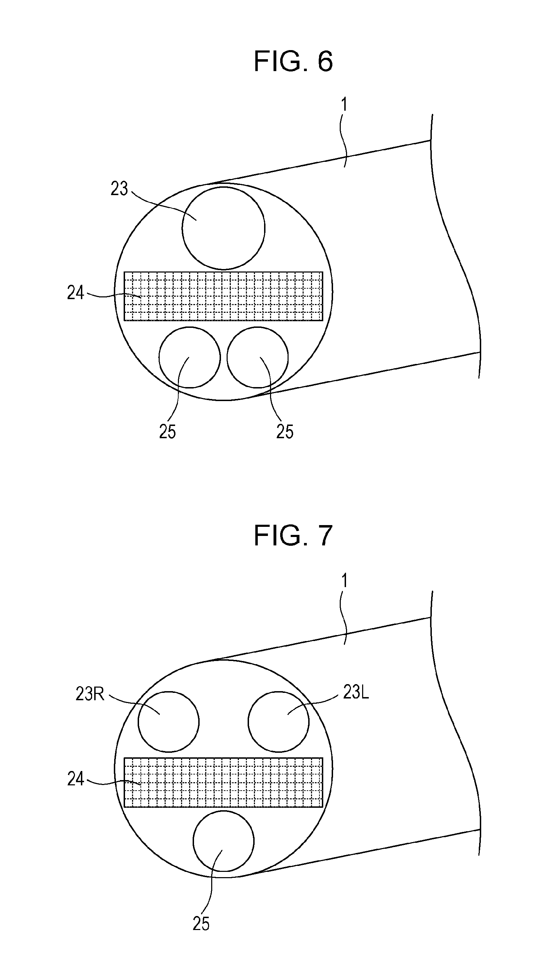

The endoscope tip 1 of the second embodiment is illustrated in Fig. 6. As described above, the observing portion 23 includes a lens, and a charge-coupled device (CCD) image sensor or a semiconductor image sensor, such as a complementary metal oxide semiconductor (CMOS) image sensor.

The illuminating-light exit portion 24 is formed by an end face of an optical fiber bundle. The endoscope tip 1 includes insertion channels 25 serving as openings for insertion of treatment tools or the like.

For better illumination control, the imaging distance (or object distance) for the objective lens serving as the observing portion 23 may be in the range of 2 cm to 10 cm. The control device 210 (see Fig. 5) includes a memory and an image-signal processing unit, such as those illustrated in the functional block diagram of Fig. 1.

The light source 130 may be a high-pressure discharge tube having a high intensity, such as a high-intensity lamp (e.g., a xenon lamp, a metal halide lamp, or a halogen lamp) or a solid light-emitting element (e.g., an inorganic LED or an organic LED).

Light emitted from a lamp, which serves as the light source 130, is guided through the light guide (not shown) to the endoscope tip 1 and exits from the illuminating-light exit portion 24.

The light guide is formed by a plurality of optical fiber bundles. The light guide may be configured such that the distribution of light incident onto the optical fiber bundles is an illumination distribution.

A limiter is provided between the light source 130 and the illuminating-light exit portion 24. The limiter is configured to limit illuminating light from a plurality of lenses and the light source 130.

For example, the limiter may be an electronic optical shutter, such as a liquid crystal element having 8 x 32 liquid crystal pixels arranged in a two-dimensional (8 x 32) matrix.

For a greater degree of freedom, an electronic element, such as a liquid crystal element, may be used as the limiter. Alternatively, a mechanical device, such as an aperture mechanism, may be used as the limiter.

If an LED array formed by many LEDs is used as the light source 130, light having a non-uniform emission intensity distribution can be generated by turning on some of many emission points forming the LED array, or by varying the intensity of a plurality of adjacent emission points. This allows setting of any illumination distribution for an object.

A light-emitting element array, such as an LED array, may be used as the illuminating-light exit portion 24. In this case, the light-emitting element array serving as the illuminating-light exit portion 24 is driven by an electrical signal from the control device 210, so that light having a desired emission intensity distribution is generated. This eliminates the need for a long optical fiber bundle.

The light emitting region of the LED array is located in the endoscope tip 1. The emission intensity of the LED array is controlled by an electric illumination control circuit in the control device 210. The light emitting region may be divided into different parts and located in multiple places in the endoscope tip 1. A light exit surface formed by the LED array may be located at the end face of the endoscope tip 1.

A treatment tool is a tool used by the operator to treat an affected area (i.e., a region of interest) while observing an object with the endoscope. Examples of the treatment tool include a cutting tool for cutting an affected area, an irradiating tool for irradiating an affected area with laser serving as light for treatment, and a suturing tool for suturing an affected area.

Visible light may be used as illuminating light in the present disclosure. The color of emission is not particularly limited, but white light may be used. Narrowband light of a specific color may be used. For fluorescent observation of an object, or for measurement of a temperature distribution of an object, light (e.g., ultraviolet or infrared light) having a wavelength band outside that of visible light may be used as illuminating light.

Accordingly, the light source used in the present disclosure may be a light source that generates light having the same spectral characteristics as those of illuminating light, a light source that generates excitation light for generating fluorescent observation light, or a light source that generates light having spectral characteristics including a wavelength of illuminating light so that necessary illuminating light can be obtained through various filters.

For fluorescent observation, the observing portion 23 may include a filter, which selectively transmits light of only a specific wavelength. After passing through the filter, light having a wavelength outside that of fluorescence to be observed is attenuated. This makes it easier to observe the state of the object.

As described above, when the relative position of the observing portion 23 and the object is changed and this causes a change in brightness distribution of the object, it is possible to correct the illumination distribution of illuminating light on the basis of image information captured.

If this is done automatically, a region of interest (i.e., object) can be observed and treated smoothly. In particular, an endoscope system that performs stereoscopic display can provide improved visibility, and an endoscope system that treats a region of interest can easily perform appropriate treatment.

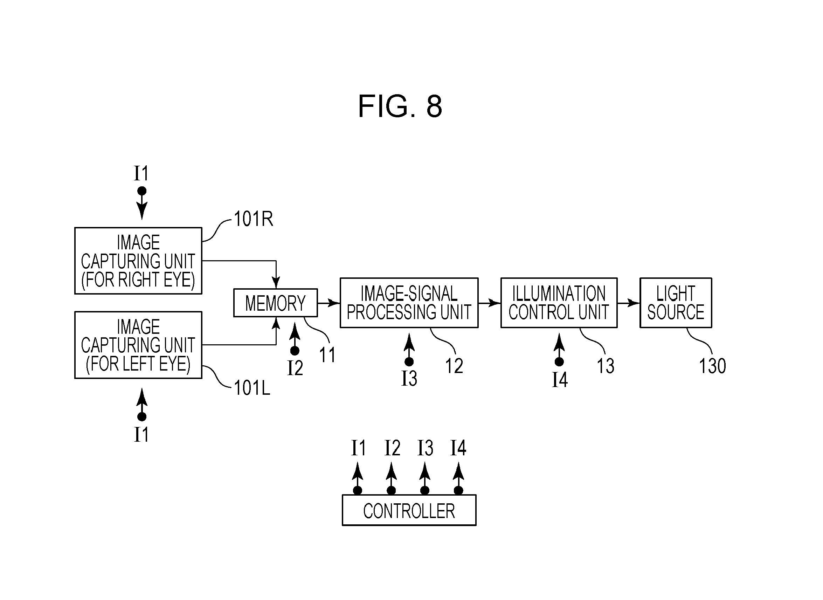

Figs. 7 and 8 are diagrams for explaining an endoscope according to a third embodiment disclosed herein. The endoscope of the third embodiment is for stereoscopically viewing an object. The endoscope tip 1 serving as an insertion portion of a main body of the stereoscopic endoscope includes a pair of observing portions 23R and 23L, the illuminating-light exit portion 24, and the insertion channel 25. The endoscope tip 1 does not necessarily need to include two observing portions, and may include three or more observing portions.

Although both the observing portions 23R and 23L are circular in Fig. 7, they may have different shapes for optimization of each of the observing portions 23R and 23L.

As illustrated in Fig. 8, the functional block of the present embodiment is substantially the same as that of Fig. 1, except that there are a plurality of image capturing units for stereoscopic viewing, and that a display controller performs image signal processing for stereoscopic display. An image capturing unit 101R for right eye and an image capturing unit 101L for left eye in Fig. 8 correspond to the observing portion 23R and the observing portion 23L in Fig. 7, respectively.

Signal processing for image recognition in the image-signal processing unit 12 is the same as steps S1 to S9 of Fig. 3. In the present embodiment, however, for optimizing the illumination distribution for stereoscopic viewing, a difference in illumination for the three-dimensional structure may be emphasized in the illumination distribution.

An optimal illumination distribution for stereoscopic viewing is one which is optimal when an object is viewed from the pair of observing portions 23R and 23L, not from only one of the observing portions 23R and 23L. That is, when an object is illuminated with an illumination distribution optimal for stereoscopic viewing, the illumination distribution does not have to be optimal for viewing from one of observing portions 23R and 23L.

In the present embodiment, once the light quantity distribution of illuminating light for illuminating an object is optimized, even when the brightness distribution of the object is changed by slightly changing the orientation, rotational angle, or position of the endoscope tip, the light quantity distribution of the illuminating light can be automatically corrected.

In the present disclosure, where a state of illumination is changed on the basis of information of an image previously captured, an object can be easily observed with an endoscope even when the orientation or position of the endoscope is changed by moving or rotating the endoscope.

While the present invention has been described with reference to exemplary embodiments, it is to be understood that the invention is not limited to the disclosed exemplary embodiments. The scope of the following claims is to be accorded the broadest interpretation so as to encompass all such modifications and equivalent structures and functions.
An endoscope has an illuminating-light exit portion (24) that allows illuminating light for illuminating an object to exit, and an observing portion (23) that captures an image of the object. The endoscope includes changing means (13) configured to change a light quantity distribution of the illuminating light. The changing means changes the light quantity distribution of the illuminating light based on information of the image captured by the observing portion.

## Claims

1. An endoscope having an illuminating-light exit portion (24) that allows illuminating light for illuminating an object to exit, and an observing portion (23) that captures an image of the object, the endoscope comprising:
changing means (13) configured to change a light quantity distribution of the illuminating light,
wherein the changing means changes the light quantity distribution of the illuminating light based on information of the image captured by the observing portion.

2. The endoscope according to Claim 1, further comprising feature-point extracting means (12) configured to extract feature points of the image based on the information of the image captured by the observing portion,
wherein the light quantity distribution of the illuminating light is changed based on information of movement of the feature points.

3. The endoscope according to Claim 2, further comprising tracking means configured to track the feature points,
wherein the light quantity distribution of the illuminating light is controlled based on information of the tracked feature points.

4. The endoscope according to any one of Claims 1 to 3, wherein the endoscope has a plurality of observing portions (23R, 23L), the endoscope further comprising a circuit configured to process image signals of images of the object captured by the plurality of observing portions, and generate an image signal for stereoscopic display.

5. The endoscope according to any one of Claims 1 to 4, wherein the changing means changes the light quantity distribution of the illuminating light based on setting information set by an operator.

6. The endoscope according to any one of Claims 1 to 5, wherein the observing portion includes a filter that selectively transmits light of a specific wavelength.

7. The endoscope according to any one of Claims 1 to 6, further comprising an insertion channel (25) configured to allow insertion of a treatment tool.

8. The endoscope according to any one of Claims 1 to 7, wherein the illuminating-light exit portion includes light generating means that generates light having a non-uniform light quantity distribution; and
the light generating means is at least one electronic element being one of a light modulating element, an electrochromic element, and a light-emitting element array.

9. The endoscope according to any one of Claims 1 to 7, wherein the illuminating-light exit portion includes light generating means that generates light having a non-uniform light quantity distribution; and
the light generating means is at least one mechanical mechanism being either an aperture or a shutter.

10. The endoscope according to any one of Claims 1 to 9, further comprising an optical fiber configured to guide light from a light source (130) to the illuminating-light exit portion.

11. The endoscope according to any one of Claims 1 to 10, wherein the observing portion includes a semiconductor image sensor.

12. The endoscope according to any one of Claims 1 to 10, further comprising an optical fiber configured to guide light from the observing portion to a semiconductor image sensor.

13. An endoscope having an illuminating-light exit portion (24) that allows illuminating light for illuminating an object to exit, and an observing portion (23) that captures an image of the object, the endoscope comprising:
changing means (13) configured to change a light quantity distribution of the illuminating light,
wherein the endoscope has a first illuminating mode in which the object is illuminated with illuminating light having a first light quantity distribution when a tip (1) of the endoscope is located at a first position, and a second illuminating mode in which the object is illuminated with illuminating light having a second light quantity distribution different from the first light quantity distribution when the tip is located at a second position that is different from the first position.

14. An endoscope system comprising:
a display device (22) configured to display an image;
and
an endoscope configured to acquire the image to be displayed on the display device,
wherein the endoscope is the endoscope according to any one of Claims 1 to 13.

15. A method for illuminating an object with an endoscope having an illuminating-light exit portion (24) that allows illuminating light for illuminating the object to exit and an observing portion (23) that captures an image of the object, the method comprising:
illuminating the object with illuminating light having a first light quantity distribution when a tip (1) of the endoscope is located at a first position; and
illuminating the object with illuminating light having a second light quantity distribution different from the first light quantity distribution when the tip is located at a second position that is different from the first position.
